# EUROPÄISCHE PATENTSCHRIFT

(11) **EP 1 712 197 B1**
(45) Veröffentlichungstag und Bekanntmachung des Hinweises auf die Patenterteilung: **14.01.2009**
(21) Anmeldenummer: 05025432.5
(22) Anmeldetag: 22.11.2005
(51) Int. Cl.: A61B 17/80, A61B 17/17

(54) **Knochenplatte**
Bone plate
Plaque d'ostéosynthèse

(30) Priorität: 11.04.2005 DE 102005017679; 13.07.2005 DE 102005033861
(43) Veröffentlichungstag der Anmeldung: 18.10.2006
(73) Patentinhaber: AAP Implantate AG, 12099 Berlin (DE)
(72) Erfinder: Ahrens, Uwe, 10179 Berlin (DE); Bakhshi, Majid, 12053 Berlin (DE); Fisher, Hans-Joachim Dr., 12277 Berlin (DE)
(74) Vertreter: von Eichel-Streiber, Caspar

(56) Entgegenhaltungen:
- WO-A-01/54601
- US-A- 5 709 686

## Beschreibung

Die Erfindung betrifft eine Knochenplatte gemäß dem Oberbegriff des Patentanspruchs 1.

Knochenplatten sind beispielsweise bekannt aus der CH 462375, der WO 2000/53111, der WO 2001/54601 oder der EP 0760632 B1.

Die Ausgestaltung der Löcher ist bei diesem Stand der Technik so, dass sie aus der Kombination von zwei sich überschneidenden Löchern bestehen mit unterschiedlichen Durchmessern und zu einem Durchgangsloch kombiniert sind und jede Seite des Durchgangslochs mit seinem festen Durchmesser seine eigene Funktionalität aufweist und auch getrennt voneinander betrachtet werden muss.

So ist es beispielsweise nicht möglich, auf der gewindeanteiligen Seite des Durchgangsloches eine Standard-Kortikalisschraube einzubringen, sondern nur eine Schraube mit Kopfgewinde.

Die andere Seite des Durchgangsloches, die keinen Gewindeanteil aufweist, ist nur für die Einbringung einer Standard-Kortikalisschraube geeignet, die auch mit Kompression und Winkelvariabilität appliziert werden kann.

Somit könnte man die Löcher auch völlig voneinander trennen da sie keine funktionellen Schnittpunkte aufweisen.

Dieser Nachteil zieht sich dann auch durch die gesamte Anwendung hin, denn beim Einbringen einer Standard-Kortikalisschraube muss auch darauf geachtet werden, dass man sich auch immer auf der richtigen Seite des Durchgangsloches befindet.

Auch bei der in der US 5,709,686 offenbarten Knochenplatte, die die wesentlichen Merkmale des Oberbegriffes des Patentanspruchs 1 aufweist, liegt noch eine funktionale Trennung vor. In einem oberen Segment des Langloches ist eine Führungsstruktur für eine Standardkortikalschraube vorgesehen, in einer darunter liegenden Ebene erstrecken sich Gewindezüge zum Festlegen einer Schraube mit Gewindekopf. Diese Gewindezüge reichen entsprechend nicht über die gesamte Höhe von der Unterseite bis zur Oberseite der Knochenplatte.

Ausgehend hiervon ist es die Aufgabe der Erfindung, die Einsatzmöglichkeiten einer Knochenplatte zu verbessern und deren Flexibilität in der Handhabung zu erhöhen.

Gelöst wird diese Aufgabe durch eine Knochenplatte mit den Merkmalen des Anspruchs 1.

Erfindungswesentlich ist, dass die Knochenplatte wenigstens ein Langloch aufweist, welches als durchgehendes Langloch ausgebildet ist. Unter durchgehendem Langloch soll ein solches Langloch verstanden werden, welches eine in seiner wesentlichen Kontur gleich bleibende Öffnungsweite aufweist.

So ist z.B. das in der WO 2001/5460 gezeigte Loch, welches dort als Kombination eines Langloches und eines Rundloches bezeichnet wird, kein durchgehendes Langloch im Sinne dieser Erfindung. Dort ist, insbesondere in der Darstellung von unten gut zu erkennen, dass das dort beschriebene Loch im Bereich des Überganges des länglichen Ovalloches zu dem Kreisloch nach innen in die Öffnung vorstehende Abschnitte der Wand aufweist. Diese Abschnitte verhindern insbesondere eine durchgängige Nutzung des insgesamt entstandenen Loches. Anders das erfindungsgemäß durchgehend ausgebildete Langloch, welches eine durchgehende, kombinierte Nutzung des Loches ermöglicht. Beispielsweise kann eine in das Langloch eingesetzte Knochenschraube entlang der gesamten Länge des Langloches problemlos verschoben werden.

Weiterhin erfindungswesentlich ist die Tatsache, dass in dem Langloch auf einem Teil des Innenumfanges eine Haltestruktur zum Festlegen eines Schraubenkopfes oder -halses einer Knochenschraube ausgebildet ist, die sich von der Oberseite bis zur Unterseite der Knochenplatte erstreckt. Haltestrukturen sind aus den oben zum Stand der Technik genannten Druckschriften prinzipiell bekannt. Dort sind die Haltestrukturen als Gewindezüge gebildet. Erfindungswesentlich für die vorliegende Erfindung ist dabei aber auch, dass zwischen einer Längsachse dieser Haltestruktur (dies ist regelmäßig die Achse, entlang derer.bei einer in der Haltestruktur festgelegten Knochenschraube die Schraubenlängsachse verläuft) eine Ebene, innerhalb derer sich eine Führungsstruktur des Langloches erstreckt unter einem von 90° verschiedenen Winkel schneidet. Mit anderen Worten sind die Längsachse der Haltestruktur und die Ebene der Führungsstruktur nicht orthogonal ausgerichtet, sondern gegeneinander verkippt. Diese Ausgestaltung zusammen mit der ebenfalls erfindungsgemäßen Form der Anlagelinie entsprechend einer Funktion zweiten oder höheren Grades ermöglicht es, eine Knochenschraube mit ihrem entsprechend komplementär ausgebildeten Kopf bzw. Hals in der Haltestruktur festzulegen, ohne hierfür die durchgehende Öffnungsweite des Langloches einengen zu müssen. D.h. insbesondere kann, wie in Anspruch 6 als vorteilhafte Ausgestaltung angegeben, die Haltestruktur so ausgebildet sein, dass sie einen Kopf bzw. Hals einer mit der Haltestruktur in Eingriff gelangenden Knochenschraube in einem Winkelbereich von maximal 180° wirksam umgreift. Ohne die geschilderte Neigung und Ausgestaltung der Anlagefläche würde bei einer so geringen Umgreifung der Kopf bzw. Hals der Knochenschraube in der Haltestruktur nicht fixiert werden, er würde bei entsprechenden Kräften aus der Haltestruktur gelöst werden und entlang des Langloches "weglaufen". Aus diesem Grund sehen die aus dem Stand der Technik bekannten Haltestrukturen regelmäßig eine Umgreifung des Halses bzw. Kopfes der Knochenschraube von deutlich mehr als 180° (üblicherweise mehr als 190°) vor. Eine solchermaßen weite Umgreifung unterbricht jedoch zwangsläufig das durchgängige Langloch. Entweder wird dann der vorbekannten Öffnungsstruktur eine "Einschnürung" geschaffen, oder der Bereich mit der Haltestruktur muss in seinem Durchmesser größer gewählt werden als der Durchmesser des Langloches.

Derartige Maßnahmen sind bei der erfindungsgemäßen Geometrie des Langloches nicht erforderlich.

Das mindestens eine durchgehende Langloch kann als vollwertiges Kombinationsloch aufgefasst werden. Der Begriff Kombinationsloch bezieht sich zum Einen auf die Kombination der verschiedenen Funktionen, die in ein und demselben Loch ausgeführt werden können, zum Anderen ist es in einer vorteilhaften Ausgestaltung gemäß Anspruch 2 die Kombination von einem Durchgangsloch mit Kompressionseigenschaft entlang der Längsachse des Langlochs und einer Haltestruktur im Innenbereich des Langlochs, die zusammenwirkend mit der erfindungsgemäßen Neigung zwischen der Längsachse der Halzeszruktur und der Ebene der Führungsstruktur der winkelstabilen Schraubenverriegelung im Kopf- bzw. Halsbereich dient.

Durch die vorstehend angegebene Neigung der Längsachse bezogen auf die Horizontale entsteht eine schiefe Ebene, die es ermöglicht, dass ein z.B. kugelig ausgebildeter Schraubenkopf oder eine winkelstabile Schraube mit einer zu der Haltestruktur korrespondierend gestalteten Gegenstruktur im Kopf- bzw. Halsbereich, beim Eindrehen entlang der Führungsstruktur des Langlochs rutscht und somit eine Kompression verursacht und den Frakturspalt im Knochen schließt.

Zur Förderung dieser Kompressionseigenschaft ist die Führungsstruktur des Langloches vorzugsweise als aufgeweiteter Randbereich ausgebildet, dessen Profil einer Außenkontur der eingesetzzen Schraubenköpfe entspricht. Hierbei werden insbesondere Knochenschrauben mit sphärisch geformten Köpfen bevorzugt, wobei ein umlaufender Rand des Langloches mit einer entsprechend kreisabschnittsförmig gebildeten Profilierung umgeben ist. Der Verlauf dieser Profilierung folgt der oben beschriebenen schiefen Ebene und ist entsprechend gegenüber der Plattenebene in einem Winkelbereich von größer 0° und kleiner 90° geneigt. Eine bevorzugte Neigung der Ebene der Führungsstruktur des Langloches gegenüber der Plattenebene liegt im Bereich von 20-30°, insbesondere 25°, ohne auf diesen Wert festgelegt zu sein. Bei einer solchermaßen gegenüber der Plattenebene geneigten Gestaltung der Führungsstruktur des Langloches ist die Haltestruktur bevorzugt in einem Bereich des Langloches angeordnet, in dem die Führungsstruktur den größten Abstand zu der Oberseite der Knochenplatte aufweist (Anspruch 3). Dies hat den Vorteil, dass auch eine mit einer entsprechenden Gegenstruktur versehene Knochenschraube zunächst einmal zum Erzeugen einer Kompression eingesetzt werden kann, d.h. entlang der schiefen Ebene der Führungsstruktur des Langloches gleitend in den Knochen eingeschraubt wird, bevor sie am Ende des Kompressionsweges in der Haltestruktur winkelstabil festgelegt wird.

In einer derzeit bevorzugten Ausgestaltung liegt die Längsachse der Haltestruktur im wesentlichen senkrecht zu der Plattenebene. Es ist allerdings auch denkbar, diese Achse aus der Lotrechten gekippt auszubilden. Dies könnte für bestimmte Anwendungen von Vorteil sein, wo eine winkelstabile Fixierung einer Knochenschraube im Knochen in einer solchen Lage gewünscht ist, die von der zur Knochenoberfläche gesehenen Lotrechten abweicht.

Bevorzugt ist die Haltestruktur an der Innenseite an einer Schmalseite des Langloches, insbesondere bevorzugt symmetrisch zu dessen längerer Achse, angeordnet (Anspruch 5).

Bereits oben ist ausgeführt, dass die Haltestruktur bevorzugt so ausgebildet ist, dass sie einen Kopf bzw. Hals einer mit dieser in Eingriff gelangenden Knochenschraube in einem Winkelbereich von maximal 180° wirksam umgreift. Je nach "Verkippung" zwischen der Längsachse der Haltestruktur und der Langlochebene können auch kleinere Winkelbereich genügen, um durch das dreidimensionale Zusammenwirken der Verkippung der Längsachse gegenüber der Langlochebene einerseits und der Anlagekontur der Haltestruktur andererseits auch bei einer derart geringen Umgreifung einer winkelstabile Festlegung der Knochenschraube zu ermöglichen (Anspruch 6). Hierbei ist es abhängig von der Art der Haltestruktur gegebenenfalls erforderlich, zur Schaffung eines gleitenden Überganges des Teils des Innenumfanges des Langloches ohne Haltestruktur zu dem Teil des mit der Haltestruktur versehenen Innenumfanges des Langloches einen Übergangsbereich zu schaffen (Anspruch 7). Im Fall der gemäß Anspruch 8 als Haltestruktur bevorzugten Gewindezüge besteht dieser Übergang in einer Weiterführung der Gewindezüge über einen Bereich von 180° hinaus, wobei diese Weiterführung sich parallel zu der geraden Kante des Langloches erstreckt und den Kopf bzw. Hals der Knochenschraube im Eingriff nicht mit umfasst. Dieser Bereich dient lediglich einer sauberen Überführung des mit einem entsprechenden Gegengewinde versehenen Halses bzw. Kopfes der Knochenschraube in die Gewindezüge, hat jedoch für das winkelstabile Festlegen der Knochenschraube keine Bedeutung. Letzteres wird ausschließlich durch die den Kopf bzw. Hals der Gewindeschraube um 180° umfassenden Gewindezüge bewirkt.

Bevorzugt ist der mit der Haltestrukrur versehene Teil des Innenumfanges des Langloches der Plattenmitte zugewandt (Anspruch 9). Dies ist insbesondere in Kombination mit einer Ausbildung gemäß Anspruch 3 von Vorteil. Damit kann nämlich erreicht werden, dass einer über eine Fraktur gelegten Platte die Knochenbruchstücke auf die Fraktur zugespannt und die Fraktur mithin komprimiert werden kann.

Besonders zu bevorzugen ist es, wenn die erfindungsgemäße Knochenplatte in sämtlichen Löchern mit wie oben beschriebenen Langlöchern ausgebildet ist (Anspruch 10). Dabei liegen die Langlöcher nicht nur mit ihrer längeren Achse in Richtung der Plattenlängsachse, sondern liegen auch im wesentlichen symmetrisch zu der in Plattenlängsrichtung verlaufenden Symmetrieachse der Platte.

Weitere Merkmale und Vorteile der Erfindung ergeben sich aus der nachfolgenden Beschreibung eines Ausführungsbeispiels anhand der beigefügten Zeichnungen. Dabei zeigen:
- Fig. 1: in dreidimensionaler Ansicht ein Ausführungsbeispiel einer erfindungsgemäßen Knochenplatte;
- Fig. 2: ein Beispiel für eine selbst schneidende Knochenschraube mit Standard-Kugelkopf;
- Fig. 3: ein Beispiel einer selbst schneidenden Knochenschraube mit an dem Schraubenkopf ausgebildetem, sphärischen Gewinde;
- Fig. 4: eine Schnittdarstellung durch die Knochenplatte gemäß Fig. 1 mit schematisch eingezeichnetem Verlauf des Langloches;
- Fig. 5a: bis 5c in unterschiedlichen Darstellungen die mögliche Verwendung einer Standard-Knochenschraube gemäß Fig. 2 in Zusammenhang mit der erfindungsgemäßen Knochenplatte;
- Fig. 6a: bis 6b schematisch die Verwendung einer in Fig. 3 gezeigte Knochenschraube im Zusammenhang mit der erfindungsgemäßen Knochenplatte;
- Fig. 7: eine Darstellung ähnlich der Fig. 4 mit eingezeichneten Konturlinien;
- Fig. 8: eine schematische Darstellung zur Erläuterung der Funktion der erfindungsgemäßen Knochenplatte im Zusammenwirken mit einer Knochenschraube gemäß Fig. 3;
- Fig. 9: in vergrößerter Darstellung einen Abschnitt eines Langloches der erfindungsgemäßen Knochenplatte mit Gewindestruktur;
- Fig. 10: eine Ansicht der erfindungsgemäßen Knochenplatte von unten;
- Fig. 11: eine Ansicht der erfindungsgemäßen Knochenplatte mit für das Bohren eines Schraubenloches im Knochen aufgesetzter Bohrhülse; und
- Fig. 12: eine ähnliche Ansicht wie Fig. 11 jedoch mit einer alternativen Variante einer Bohrhülse.

In den Figuren ist schematisch ein Ausführungsbeispiel einer erfindungsgemäßen Knochenplatte gezeigt und allgemein mit 1 bezeichnet. Das gezeigte Ausführungsbeispiel ist keinesfalls maßstabsgerecht, sondern dient lediglich einer prinzipiellen Erläuterung.

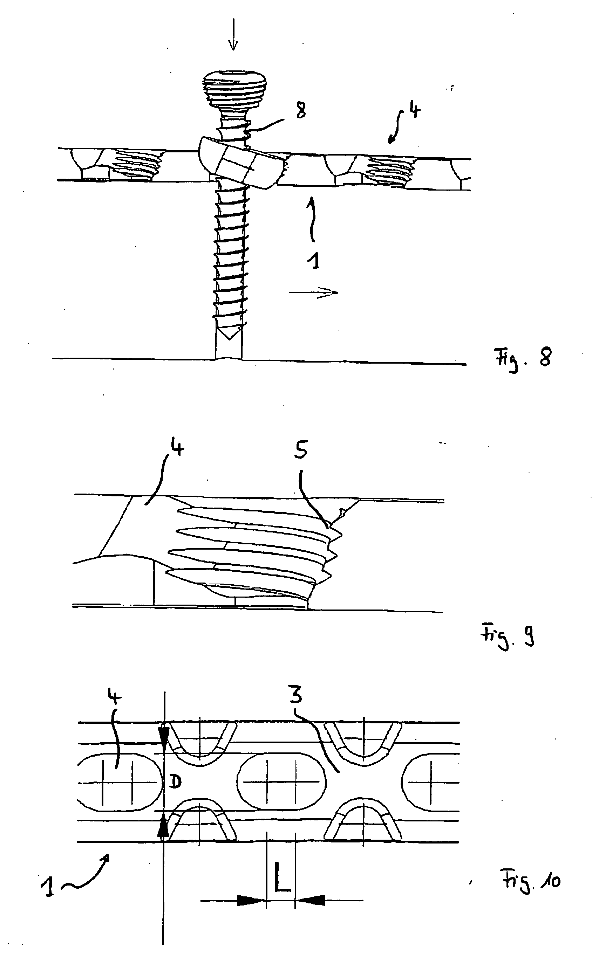

Die erfindungsgemäße Knochenplatte 1 hat eine Oberseite 2 und eine im Anwendungsfall dem fragmentierten Knochen zugewandte Unterseite 3 (vgl. Fig. 10). In der Knochenplatte sind entlang ihrer Längsachse ausgerichtete Langlöcher 4 eingebracht. Diese Langlöcher 4 weisen jeweils auf ihrer der Plattenmitte zugewandten Stirnseite eine Haltestruktur in Form eines Gewindes 5 auf. Anstelle der in diesem Ausführungsbeispiel gewählten Haltestruktur in Form eines Gewindes könnten andere Arten von Haltestrukturen gewählt werden, beispielsweise mit entsprechenden Haltezapfen zusammenwirkende Haltenuten oder dgl.

Die Langlöcher 4 sind durchgängig von der Oberseite 2 zu der Unterseite 3 der Platte und weisen in ihrem der Oberseite 2 zugewandten Abschnitt eine Führungsstruktur in Form einer Führungsfläche 6 auf. Die Führungsfläche 6 weist eine Kontur mit einem Profil f(x) auf. F(x) ist dabei eine Funktion 2. oder höheren Grades, in diesem Ausführungsbeispiel ein Teilkreisabschnitt.

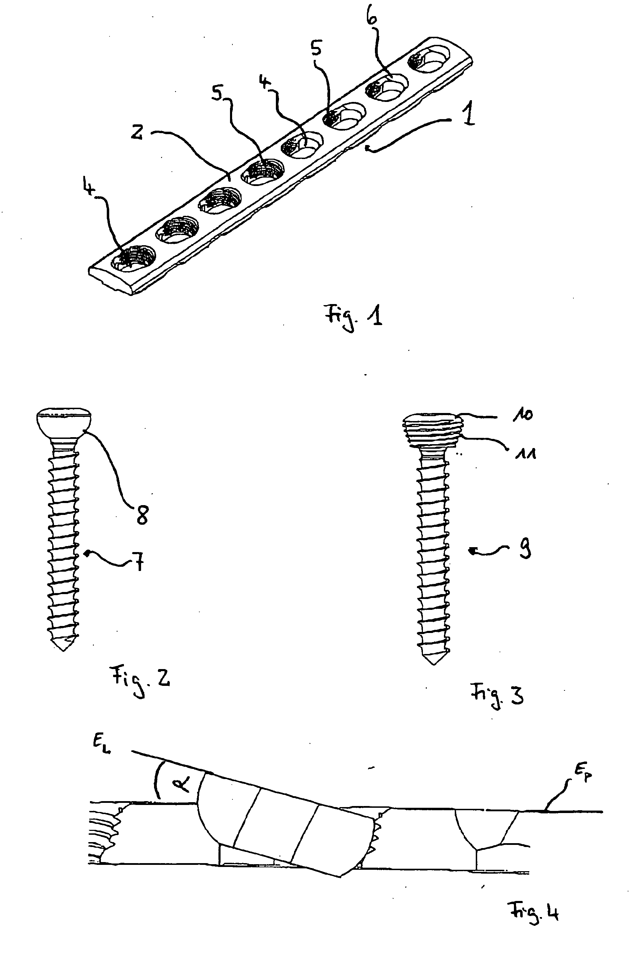

Der Übergang zwischen dem Profil und dem "geraden Abschnitt" des Langloches 4 bildet eine Ebene E_{L}, die geneigt zu einer Plattenebene Ep verläuft. Dieses ist besonders gut in Fig. 4 zu erkennen, in welcher die Kontur des Langloches mit der Führungsfläche 6 als schematisch dargestelltes "badewannenähnliches" Objekt eingezeichnet ist. Zwischen der Ebene Eₚ und der Ebene E_{L} ist ein Neigungswinkel α ausgebildet, welcher im gezeigten Ausführungsbeispiel 25° berrägt.

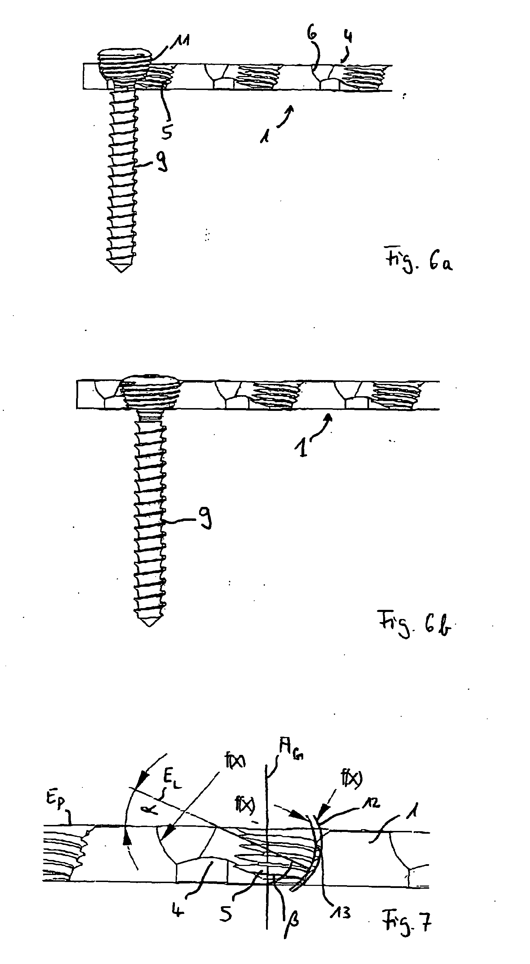

Die Gewindestruktur 5 bestimmt eine Gewindelängsachse A_{G}, welches die Achse ist, um welche ein in das Gewinde 5 eingedrehtes Gegengewinde rotiert. Diese Gewindelängsachse A_{G} ist, wie in Fig. 7 gut zu erkennen ist, gegenüber der Ebene E_{L} um einen Winkel β geneigt. Mit der Plattenebene E_{P} schließt die Gewindelängsachse A_{G} einen rechten Winkel ein.

Diese geometrischen Verhältnisse werden in ihrer Bedeutung und in ihrem Zusammenwirken nachfolgend noch näher erläutert werden.

In den Figuren 2 und 3 sind Darstellungen von zwei Knochenschrauben 7 bzw. 9 gegeben. Die in Fig. 2 dargestellte Knochenschraube 7 hat einen Standard-Schraubenkopf 8. Dieser ist glatt ausgebildet mit einer im wesentlichen sphärischen Kontur.

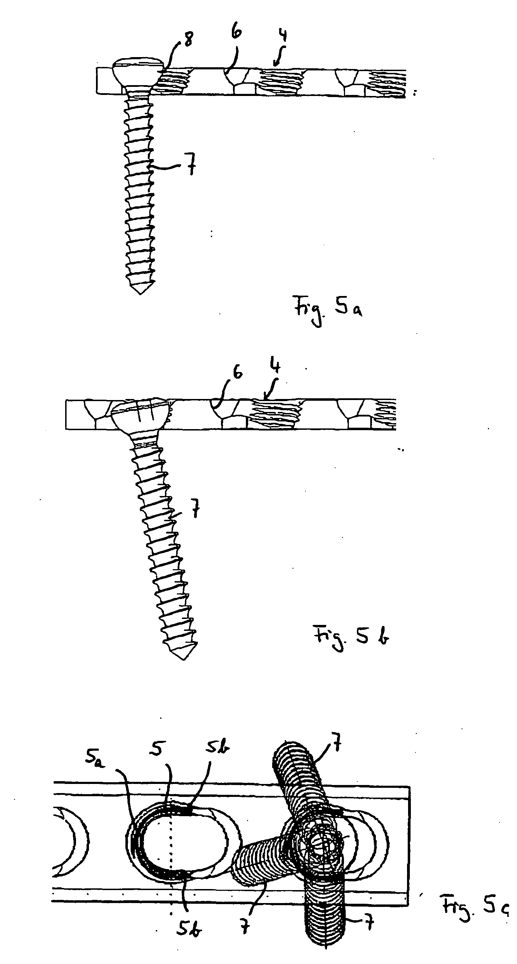

Der Schraubenkopf 10, der in Fig. 3 dargestellten Knochenschraube 9 ist ebenfalls mit einer sphärischen Kontur gebildet, weist jedoch ein sphärisches Gewinde 11 auf. Beide Knochenschrauben 7 und 9 sind zur Verwendung mit der erfindungsgemäßen Knochenplatte 1 gedacht. Dies wird in den Figuren 5a bis 5c bzw. 6a und 6b zunächst einmal erläutert. Die sphärische Außenkontur des Schraubenkopfes 8 der Knochenschraube 7 entspricht in ihrem Radius dem Radius des Profils f(x) der Führungsfläche 6. Damit kann die wie in Fig. 5a gezeigt in das Langloch 4 eingesetzte Knochenschraube 7 entlang der Führungsfläche 6 und damit entlang der gegenüber der Plattenebene E_{P} geneigten, schiefen Ebene (Ebene E_{L}) geführt verschoben werden. Auch die äußere Kontur der Gewindezüge (der Zähne) des Gewindes 5 ist mit dem Profil f(x) gebildet. Entsprechend kann auch hier der Kopf 8 der Knochenschraube 7 geführt anliegen. Aufgrund einer fehlenden Verankerung des Kopfes 8 der Knochenschraube 7 lässt sich diese Knochenschraube 7 in jeder Position sowohl in Richtung der Längsachse der Platte als auch seitlich dazu neigen und zwar um Winkel von etwa 20° gemessen zu der Lotrechten der Plattenebene E_{P} (vgl. Fig. 5c).

Auf diese Weise kann die Knochenschraube 7 für die Knochenplatte 1 zum Komprimieren eingesetzt werden. Die Knochenschraube 7 wird hierzu in den Knochen eingeschraubt und "wandert" beim Anziehen entlang der schiefen Ebene E_{L} nach unten. Dadurch bringt sie auf die Platte eine Kraft auf, die eine parallel zu der Plattenebene Ep verlaufende- Komponente aufweist und somit eine Kompressionswirkung ausübt.

Auf ähnliche Weise kann auch eine mit einem Gewinde 11 am Schraubenkopf 10 versehene Knochenschraube 9 in dem Langloch geführt bewegt werden. Auch hier dient die Führungsfläche 6 der Führung des Schraubenkopfes 10, dessen Radius der Kontur des Profils f(x) entspricht. Im Unterschied zu der Knochenschraube 7 mit gewindelosem Schraubenkopf 8 kann die Knochenschraube 9 jedoch nach einem Eingriff des Gewindes 11 am Schraubenkopf 10 mit dem Gewinde 5 auf der Innenseite des Langloches 4 in ihrem Winkel nicht mehr verschoben werden. Ihre Längsachse ist dann festgelegt parallel zu der Gewindelängsachse A_{G} und mithin lotrecht zu der Plattenebene E_{P}. Dieser Halteeffekt wird wie nachfolgend geschildert bewirkt.

Das auf der Innenseite des Langloches 4 stirnseitig angeordnete Gewinde 5 weist zwei durch die innen liegenden bzw. vorstehenden Begrenzungslinien der Gewindelinien gebildete Konturen 12 bzw. 13 auf. Diese Konturen verlaufen jeweils in den Profilen f (x), welche hier teilkreisförmige Profile sind. Allerdings sind die Konturen 12 (Außenkontur) und 13 (Innenkontur) gegeneinander "versetzt", haben mithin versetzte Ursprünge. Grundsätzlich ist es auch möglich, die Profile f(x) der Konturen 12 und 13 nach unterschiedlichen Funktionen zu bilden. Das Gewinde 5 umfasst den Schraubenkopf 10 in einer Endstellung lediglich um 180° bzw. liegt nur über einen solchen Winkel wirksam an dem Gewinde 11 des Schraubenkopfes 10 an. Dadurch, dass sich zwischen der Gewindelängsachse A_{G} und der Ebene E_{L} ein Winkel β bildet, wird hier ein Klemmeffekt erzielt, der auch bei einer solch geringen Umgreifung einen sicheren Halt des Schraubenkopfes 10 bewirkt. In Fig. 5c besonders gut zu erkennen ist, dass das Innengewinde 5 grob zwei Abschnitte aufweist, einen wirksamen Bereich 5a, der sich bei in dem Gewinde festsitzender Schraube um etwa 180° um den Schraubenkopf 10 herum erstreckt sowie Gewindeausläufe 5b, die gerade verlaufen, entlang dem seitlichen Abschnitt des Langloches auf der in Fig. 10 mit L bezeichneten Strecke.

Diese Ausläufe 5b haben keine Haltefunktion, sondern dienen lediglich einer sicheren Überführung des mit dem Gewinde 11 versehenen Schraubenkopfes 10 in den wirksamen Bereich 5a des Gewindes 5.

Für die Fertigung der Langlöcher in der erfindungsgemäßen Knochenplatte 1 wird wie folgt vorgegangen: Zunächst wird ein Langloch mit einem CNC-Fräser in die Platte eingefräst. Dabei wird anschließend von dem CNC-Fräser die Kontur der Führungsfläche eingebracht, wobei hierdurch die Neigung der schiefen Ebene E_{L} gegenüber der Plattenebene E_{P} festgelegt wird. Die Neigung wird so gelegt, dass sie stets in Richtung der Plattenmitte abwärts geneigt verläuft. In einem anschließenden Arbeitsgang wird an dem der Plattenmitte zugewandten, nach unten geneigten Ende des Langloches 4 mit einem weiteren Fräser ein Gewindeabschnitt eingefräst. Dieser wird so eingestellt, dass der Gewindeabschnitt in seiner Längsachse gegenüber der Langlochebene E_{L} geneigt ist, diese also unter einem von 90° verschiedenen Winkel schneidet. Bei dieser Bearbeitung werden automatisch die versetzten Konturen 12 und 13 erzeugt. Die innen gelegene Kontur 13 ist die durch den beim Herstellen der Führungsfläche verwendeten Fräsvorgang erzeugte Kontur, die Kontur 12 ergibt sich durch den Gewindefräsprozess. Beim Fräsen des Gewindes werden neben dem wirksamen Bereich 5a auch die Ausläufe 5b mit erzeugt.

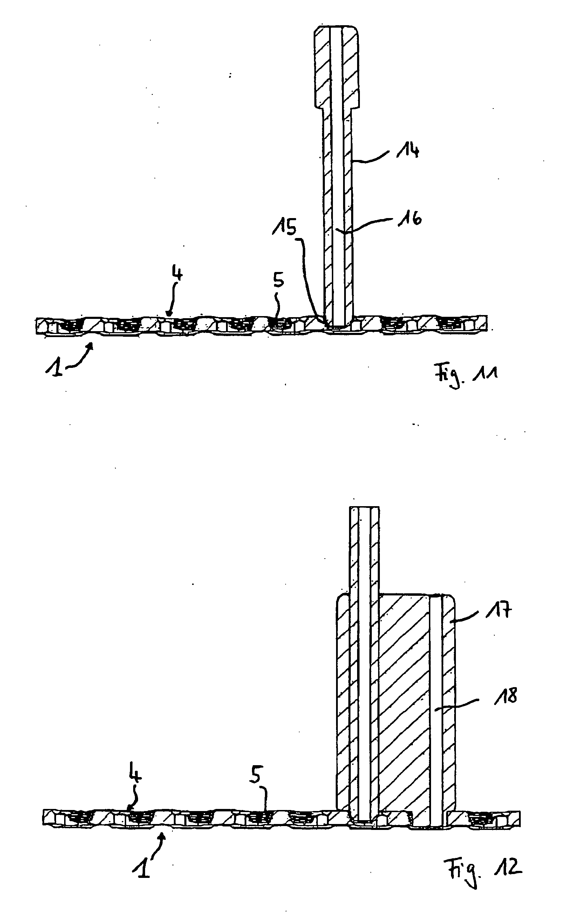

In den Figuren 11 und 12 ist dargestellt, wie für die Operation zum Einbau der Knochenplatte Bohrhülsen mit der Knochenplatte verbunden sind. Die in Figur 11 dargestellte Situation zeigt eine einfache Bohrhülse 14, die mit einem an ihrem unteren Ende gebildeten sphärischen Gewinde 15 in das Gewinde 5 auf der Innenseite des Langloches 4 eingeschraubt wird. Die Bohrhülse 14 gibt in ihrem Innern einen Bohrkanal 16 vor, der durch die passgerechte Aufnahme des Gewindes 15 der Bohrhülse 14 entlang der Achse A_{G} verläuft. Die Bohrhülse 14 dient als Hilfsmittel, um beim Anbohren des mit der Knochenplatte 1 zu versorgenden Knochens sicherzustellen, dass das Bohrloch lotrecht zu der Knochenplatte 1 verläuft und so eine Knochenschraube 9 winkelstabil so in den Knochen geschraubt werden kann, dass ihr Schraubenkopf 10 mit dem Gewinde 11 problemlos in das Gewinde 5 eingreift.

In Figur 12 ist eine Variante gezeigt, in der auf eine Bohrhülse 14 eine weitere Bohrhülse 17 so aufgesetzt ist, dass der Bohrkanal 18 dieser Bohrhülse 17 exakt parallel zu der Achse A_{G} liegt. Mit dieser Bohrhülse 17 können in den Knochen Schraubenlöcher gebohrt werden, die die oben beschriebene Doppelfunktion einer Knochenschraube 9 erlauben. Die Knochenschraube 9 wird dabei in einem solchen, lotrechten Winkel in den Knochen geschraubt, der es ihr erlaubt, zunächst für eine Kompression entlang der schiefen Ebene E_{L} der Führungsfläche 6 zu gleiten und anschließend mit dem Gewinde 11 an ihrem Schraubenkopf 10 sicher und unverkantet in das Gewinde 5 auf der Innenseite des Langloches 4 einzugreifen, um letztlich in ihrem Winkel fixiert zu werden. Dieser Vorgang erlaubt letztlich die gleichzeitige Kompression und Winkelfixierung mit nur einer Schraube in einem Arbeitsgang.

Die gezeigten Bohrhülsen sind rein exemplarisch, und es gibt verschiedene Wege, dieselben Ergebnisse mit anders konstruierten Bohrhülsen zu erzielen.

Aus dieser Beschreibung des Ausführungsbeispiels ist deutlich geworden, welche Vorteile die erfindungsgemäße Ausbildung einer Knochenplatte hat. Das Ausführungsbeispiel ist rein beschreibend. Insbesondere ist der Schutzumfang der nachfolgenden Patentansprüche nicht auf dieses Ausführungsbeispiel beschränkt.

### Bezugszeichenliste

- 1: Knochenplatte
- 2: Oberseite
- 3: Unterseite
- 4: Langloch
- 5: Gewinde
- 5a: wirksamer Bereich
- 5b: Auslauf
- 6: Führungsfläche
- 7: Knochenschraube
- 8: Schraubenkopf
- 9: Knochenschraube
- 10: Schraubenkopf
- 11: Gewinde
- 12: Kontur
- 13: Kontur
- 14: Bohrhülse
- 15: Gewinde
- 16: Bohrkanal
- 17: Bohrhülse
- 18: Bohrkanal
- A_{C}: Gewindelängsachse
- D: Durchmesser
- E_{L}: Ebene
- E_{P}: Plattenebene
- f (x): Profil
- L: Länge
- α: Winkel
- β: Winkel

## Patentansprüche

1. Knochenplatte mit einer für die Anlage am Knochen ausgebildeten Unterseite (3) und einer vom Knochen abgewandten Oberseite (2) sowie mit einer Mehrzahl von im wesentlichen entlang der Plattenlängsachse vorgesehenen Löchern, durch die zur Verankerung Knochenschrauben (7, 9) mit dem Knochen in Eingriff bringbar sind, wobei wenigstens eines der Löcher als durchgängiges Langloch (4) ausgebildet ist, mit der längeren Achse in Richtung der Plattenlängsachse, wobei das Langloch (5) der Oberseite (2) zugewandt eine Führungsstruktur (6) zur Führung eines Schraubenkopfes (8, 10) der Knochenschraube (7, 9) aufweist, und wobei auf einem Teil des Innenumfanges des Langloches (4) eine Haltestruktur (5) zum Festlegen eines Schraubenkopfes (10) oder -halses einer Knochenschraube (9) ausgebildet ist, welche eine Längsachse (A_{G}) definiert, wobei die Haltestruktur (5) in einer Ebene ihrer Längsachse eine Anlagelinie (f(x)) für den Schraubenkopf (10) oder -hals der Knochenschraube (9) aufweist, welche durch eine Funktion zweiten oder höheren Grades beschrieben ist, vorzugsweise eine teilkreisförmige Anlagelinie, wobei die Längsachse (A_{G}) der Haltestruktur (5) eine Ebene (E_{L}), innerhalb derer sich die Führungsstruktur (6) erstreckt, unter einem von 90° verschiedenen Winkel (β) schneidet, wobei der mit der Haltestruktur (5) versehene Teil des Innenumfanges zugleich Bestandteil der Führungsstruktur (6) ist, **dadurch gekennzeichnet, dass** sich die Haltestruktur (5) von der Oberseite bis zur Unterseite der Knochenplatte erstreckt.

2. Knochenplatte nach Anspruch 1, **dadurch gekennzeichnet, dass** die Ebene (E_{L}), innerhalb derer sich die Führungsstruktur (6) erstreckt, um einen Winkel (α) von größer 0° und kleiner 90° gegenüber der Plattenebene (Eₚ) geneigt ist.

3. Knochenplatte nach Anspruch 2, **dadurch gekennzeichnet, dass** die Haltestruktur (5) in einem Bereich des Langloches (4) angeordnet ist, in dem die Führungsstruktur (6) den größten Abstand zu der Oberseite (2) der Knochenplatte (1) aufweiset.

4. Knochenplatte nach einem der vorhergehenden Ansprüche, **dadurch gekennzeichnet dass** die Längsachse (A_{G}) der Haltestruktur (5) im wesentlichen senkrecht zu der Plattenebene (E_{P}) liegt.

5. Knochenplatte nach einem der vorhergehenden Ansprüche, **dadurch gekennzeichnet, dass** die Haltestruktur (5) an der Innenseite an einer Schmalseite des Langloches (4), vorzugsweise symmetrisch zu dessen längerer Achse angeordnet ist.

6. Knochenplatte nach einem der vorhergehenden Ansprüche, **dadurch gekennzeichnet, dass** die Haltestruktur (5) so ausgebildet ist, dass sie einen Kopf (10) bzw. Hals einer mit dieser in Eingriff gelangenden Knochenschraube (9) in einem Winkelbereich (5a) von maximal 180° wirksam umgreift.

7. Knochenplatte nach Anspruch 6, **dadurch gekennzeichnet, dass** ein Übergangsbereich (5b) vorgesehen ist, zur Schaffung eines gleitenden Überganges des Teils des Innenumfanges des Langloches (4) ohne Haltestruktur zu dem Teil des mit der Haltestruktur (5) versehenen Innenumfanges des Langloches (4).

8. Knochenplatte nach einem der vorhergehenden Ansprüche, **dadurch gekennzeichnet, dass** die Haltestruktur (5) durch Gewindezüge gebildet ist.

9. Knochenplatte nach einem der vorhergehenden Ansprüche, **dadurch gekennzeichnet, dass** der mit der Haltestruktur (5) versehene Teil des Innenumfanges des Langloches (4) der Plattenmitte zugewandt ist.

10. Knochenplatte nach einem der vorhergehenden Ansprüche, **dadurch gekennzeichnet, dass** alle Löcher als Langlöcher (4) mit den anspruchsgemäßen Merkmalen ausgebildet sind.

## Claims

1. Bone plate with a lower side (3) constructed for bearing on the bone and an upper side (2) remote from the bone, as well as with a plurality of holes essentially provided along the plate longitudinal axis and through which bone screws (7, 9) can be engaged with the bone for anchoring purposes, at least one of the holes being constructed as a through elongated hole (4), with the longer axis in the direction of the plate longitudinal axis, the elongated hole (5), facing the upper side (2), having a guide structure (6) for guiding a screw head (8, 10) of the bone screws (7, 9) and where on part of the inner circumference of the elongated hole (4) is formed a retaining structure (5) for fixing a screw head (10) or neck of a bone screw (9), which defines a longitudinal axis (A_{G}), the retaining structure (5) having in a plane of its longitudinal axis a bearing line (f(x)) for the screw head (10) or neck of the bone screw (9) described by a second or higher degree function, preferably a pitch circular bearing line, the longitudinal axis (A_{G}) of the retaining structure (5) intersecting a plane (E_{L}), within which the guide structure (6) extends, under an angle (β) differing from 90°, the part of the inner circumference provided with the retaining structure (5) being at the same time part of the guide structure, **characterized in that** the retaining structure (5) extends from the upper side to the lower side of the bone plate.

2. Bone plate according to claim 1, **characterized in that** the plane (E_{L}), within which extends the guide structure (6), is inclined by an angle (α) greater than 0° and smaller than 90° with respect to the plate plane (E_{P}).

3. Bone plate according to claim 2, **characterized in that** the retaining structure (5) is located in an area of the elongated hole (4), in which the guide structure (6) has the maximum spacing from the upper side (2) of the bone plate (1).

4. Bone plate according to one of the preceding claims, **characterized in that** the longitudinal axis (Ac) of the retaining structure (5) is substantially perpendicular to the plate plane (E_{P}).

5. Bone plate according to one of the preceding claims, **characterized in that** the retaining structure (5) is positioned on the inside on a narrow side of the elongated hole (4), preferably symmetrically to its longer axis.

6. Bone plate according to one of the preceding claims, **characterized in that** the retaining structure (5) is constructed in such a way that it embraces in an effective manner a head (10) or neck of a bone screw (9) coming into engagement therewith in an angular range (5a) of max. 180°.

7. Bone plate according to claim 6, **characterized in that** there is a transition area (5b) for creating a continuous transition of the part of the inner circumference of the elongated hole (4) without the retaining structure with the part of the inner circumference of elongated hole (4) provided with the retaining structure (5).

8. Bone plate according to one of the preceding claims, **characterized in that** the retaining structure (5) is formed by threads.

9. Bone plate according to one of the preceding claims, **characterized in that** the part of the inner circumference of the elongated hole (4) equipped with the retaining structure (5) faces the centre of the plate.

10. Bone plate according to one of the preceding claims, **characterized in that** all the holes are constructed as elongated holes (4) with the features of the claims.

## Revendications

1. Plaque pour os comprenant un côté inférieur (3) formé pour l'appui sur l'os et un côté supérieur (2) opposé à l'os ainsi qu'une pluralité de trous prévus essentiellement le long de l'axe longitudinal de la plaque, par lesquels des vis pour os (7, 9) peuvent être mises en prise avec l'os pour l'ancrage, au moins l'un des trous étant conçu comme trou oblong (4) continu, avec l'axe plus long dans le sens de l'axe longitudinal de la plaque, le trou oblong (5), tourné vers le côté supérieur (2), présentant une structure de guidage (6) pour le guidage d'une tête de vis (8, 10) de la vis pour os (7, 9), et une structure de retenue (5) étant conçue sur une partie du pourtour intérieur du trou oblong (4) pour la fixation d'une tête de vis (10) ou d'un collet d'une vis pour os (9), qui définit un axe longitudinal (A_{G}), la structure de retenue (5) présentant dans un plan de son sens longitudinal une ligne d'appui (f(x)) pour la tête de vis (10) ou le collet de la vis pour os (9), qui est décrite par une fonction de second degré ou de degré supérieur, de préférence une ligne d'appui en forme de cercle partiel, l'axe longitudinal (A_{G}) de la structure de retenue (5) coupant un plan (E_{L}), à l'intérieur duquel s'étend la structure de guidage (6), sous un angle (β) différent de 90°, la partie, dotée de la structure de retenue (5), du pourtour intérieur faisant partie en même temps de la structure de guidage (6), **caractérisée en ce que** la structure de retenue (5) s'étend depuis le côté supérieur jusqu'au côté inférieur de la plaque pour os.

2. Plaque pour os selon la revendication 1, **caractérisée en ce que** le plan (E_{L}), à l'intérieur duquel s'étend la structure de guidage (6), est incliné d'un angle (α) supérieur à 0 et inférieur à 90 ° par rapport au plan de plaque (Eₚ).

3. Plaque pour os selon la revendication 2, **caractérisée en ce que** la structure de retenue (5) est disposée dans une zone du trou oblong (4) dans laquelle la structure de guidage (6) présente la plus grande distance au côté supérieur (2) de la plaque pour os (1).

4. Plaque pour os selon l'une quelconque des revendications précédentes, **caractérisée en ce que** l'axe longitudinal (A_{G}) de la structure de retenue (5) est disposée sensiblement perpendiculairement au plan de plaque (E_{P}).

5. Plaque pour os selon l'une quelconque des revendications précédentes, **caractérisée en ce que** la structure de retenue (5) est disposée sur le côté intérieur sur un côté étroit du trou oblong (4), de préférence symétriquement par rapport à l'axe plus long du trou.

6. Plaque pour os selon l'une quelconque des revendications précédentes, **caractérisée en ce que** la structure de retenue (5) est constituée de telle sorte qu'elle entoure d'une façon efficace une tête (10) ou un collet d'une vis pour os (9) venant en prise dans une plage angulaire (5a) maximale de 180°.

7. Plaque pour os selon la revendication 6, **caractérisée en ce que** lorsqu'il est prévu une zone de transition (5b), pour la création d'une transition glissante de la partie du pourtour intérieur du trou oblong (4) sans structure de retenue avec la partie du pourtour intérieur, dotée de la structure de retenue (5), du trou oblong (4).

8. Plaque pour os selon l'une quelconque des revendications précédentes, **caractérisée en ce que** la structure de retenue (5) est formée par des rayures de filetage.

9. Plaque pour os selon l'une quelconque des revendications précédentes, **caractérisée en ce que** la partie, dotée de la structure de retenue (5), du pourtour intérieur du trou oblong (4) est tournée vers le centre de la plaque.

10. Plaque pour os selon l'une quelconque des revendications précédentes, **caractérisée en ce que** tous les trous sont conçus comme des trous oblongs (4) avec les caractéristiques conformes à l'invention.
